# EUROPEAN PATENT APPLICATION

(11) **EP 3 936 120 A1**
(43) Date of publication of application: **12.01.2022**
(21) Application number: 20184227.5
(22) Date of filing: 06.07.2020
(51) Int. Cl.: A61K 9/46, A61K 9/00, A61K 9/06, A61K 9/20, A23L 2/40, A23L 2/54, A23L 3/358, A23L 29/00, A61Q 19/08

(54) **HYDROGEN-GENERATING COMPOSITIONS AND KITS**

(71) Applicant: Lehmann, Ira Yasmin, 52428 Jülich (DE); Perez, Philip, 52428 Jülich (DE)
(72) Inventor: Lehmann, Ira Yasmin, 52428 Jülich (DE)
(74) Representative: Hoffmann Eitle

(57) **Abstract**

The present invention relates to a composition or kit of compositions comprising, either in a single composition or in distinct compositions of a kit of compositions, a compound or a combination of compounds capable of generating molecular hydrogen (H₂) and comprising at least one of calcium, zinc, magnesium, and iron , and one or more active ingredients selected from the group consisting of nicotinamide mononucleotide (NMN), glutathione, hyaluronic acid and salts thereof, polyunsaturated fatty acids, salts and C1-C6 alkyl esters thereof, mono- and polyunsatured oils and fats, medium-chain triglycerides, fruits and vegetables and extracts thereof, coenzyme Q10, aloe vera and vitamins other than vitamin C. The compositions and the kits are useful as pharmaceutic preparation, cosmetic preparation or as food supplement, e.g. in order to treat or prevent signs of aging, oxidative stress and/or conditions associated therewith, such as cancer.

## Description

### TECHNICAL FIELD

The present invention relates to a composition or a kit of compositions comprising a compound capable of generating molecular hydrogen (H₂) and one or more active ingredients. The compositions or the kits can be used in medicinal and cosmetic applications or as a food supplement.

### BACKGROUND ART

In recent years, molecular hydrogen attracted significant interest in the pharmaceutical and cosmetic field. A treatment with molecular hydrogen is also considered to contribute to the well-being of a human. Molecular hydrogen is typically produced either by electrolysis of water, requiring bulky machinery and electricity to work, or by preparing aqueous solutions that are saturated with hydrogen. In respect of the letter, typically an effervescent tablet is used that comprises a compound that upon contact with water is capable of generating hydrogen. This is typically magnesium in particulate form, as such particulate magnesium is capable of generating considerable amounts of hydrogen upon contact with water (see e.g. M. Uda et al, Sci Technol Adv Mater. 2012 Apr; 13(2): 025009). Commercial effervescent tablets are for instance known under the tradenames hydronade^{™} (available from aquacentrum^{™}), Rejuvenatio^{™} n HRW, H2 Elite^{™}, and Active H2 Purative^{™}.

In the pharmaceutic and cosmetic field, as well as in the area of food supplements, furthermore compositions comprising certain active ingredients are known. For instance, cosmetics utilising a compound such as coenzyme Q 10 or Aloe Vera are in common use.

Also known are pharmaceutical compositions and food supplements containing polyunsaturated fatty acids, salts and short chain (having 1 to 6 carbon atoms) alkyl esters thereof, such as DHA and EHA and ethyl esters thereof, as these are believed to contribute to a human's well-being and health by reducing oxidative stress. Such compositions may also be used to facilitate the treatment or prevention of cardiovascular diseases.

Further known are pharmaceutical compositions and food supplements that contain fruit or vegetable extracts or vitamins or a combination of vitamins (such as vitamin A, B1, B6, B12, C, D3 or K, e.g. a combination of vitamin K2 and D3). Such compositions can be used as pharmaceuticals in case of an underlying deficiency, but are also often used as a food supplement by consumers.

### PROBLEMS TO BE SOLVED BY THE PRESENT INVENTION

Many active ingredients in food supplements or in pharmaceutical preparations are prone to oxidative decay. Such oxidative decay not only occurs at the time of preparation or storage, but also when in the hands of the consumer or patient. Also drinkable preparations for pharmaceutical applications or as food supplement may undergo an oxidation reaction upon contact with water or moisture. Oxidative decay may also occur when e.g. a food supplement is mixed with other components of the food. While this is of no major concern in e.g. of the case of vitamin C, which in itself is known to act as an antioxidant, the problem may be more pronounced for other vitamins or components of food supplements. Oxidative decay of a cosmetic components can also occur after application to a user's skin.

Hydrogen generating compositions in the form of effervescent tablets are however limited in their application, and generally are intended to only provide the user or patient with molecular hydrogen. The applicability of such compositions is thus very limited, and allows only for the hydrogen to be ingested in drinkable form. Furthermore, such hydrogen generating compositions in the form of effervescent tablets generally do not contain any other active ingredient.

It is therefore one object of the present invention to broaden the possible application of molecular hydrogen in the cosmetic field, the pharmaceutical field and the field of functional foods or food supplements.

It is another object of the present invention to minimise the oxidative decay of active ingredients to maximise delivery to the user or patient.

It is another object of the present invention to provide for food supplements and pharmaceuticals that have an improved action against oxidative stress. This would be beneficial for a variety of applications, e.g. for the treatment of diseases that are associated with oxidative stress, such as certain types of cancer. An improved action against oxidative stress may also be beneficial in the cosmetic field, as oxidative stress is considered to be responsible for signs of ageing and skin degeneration. Hence, the compositions of the present invention, respectively the kit of combinations, can also be useful as anti-aging products.

### SUMMARY OF THE INVENTION

The present invention was achieved based on the idea that the beneficial effect of molecular hydrogen in the field of pharmaceuticals, cosmetics and food supplements can be boosted by combining molecular hydrogen with other active ingredients that act against oxidative stress. Given that such other active ingredients are also prone to oxidative decay, the hydrogen not only acts as biologically active agent on its own merit, but also has the effect of acting as an antioxidant or reducing agent on the active ingredient, thereby maximising delivery of the active ingredient to a user's or patient's body. In the composition of the present invention (or in the kit of compositions of the present invention, once combined by the user or medical staff) there is therefore not merely an additive effect of the molecular hydrogen and the active ingredient, but rather an interaction/synergy between the active ingredient and hydrogen becomes possible.

The present invention includes the following aspects:
1. A composition or kit of compositions comprising, either in a single composition or in distinct compositions of a kit of compositions,
   A a compound or a combination of compounds capable of generating molecular hydrogen (H₂) and comprising at least one of calcium, zinc, magnesium, and iron , and
   B one or more active ingredients selected from the group consisting of nicotinamide mononucleotide (NMN), glutathione, hyaluronic acid and salts thereof, polyunsaturated fatty acids, salts and C1-C6 alkyl esters thereof, mono- and polyunsatured oils and fats, medium-chain triglycerides, fruits and vegetables or extracts thereof, coenzyme Q10, aloe vera and vitamins other than vitamin C.
2. Composition or kit of compositions according to aspect 1 or 2, wherein A, the compound or combination of compounds capable of generating molecular hydrogen (H₂) and comprising at least one of calcium, zinc, magnesium, and iron, comprises a compound that generates molecular hydrogen upon contact with water, or comprises a combination of compounds that by reaction with each other generate molecular hydrogen.
3. Composition or kit of compositions according to any one of aspects 1 to 3, which further comprises
   C one or more additives, preferably selected from the group consisting of antioxidants, fillers, such as starch, buffers, preferably a carbonate/bicarbonate buffer, pH adjusting agents, flavours, sugars, alkali earth salts of organic acids, and vitamin C.
4. Composition or kit of compositions according to any one of aspects 1 to 3, wherein A, the compound or a combination of compounds capable of generating molecular hydrogen (H₂) and comprising at least one of calcium, zinc, magnesium, and iron, is a combination of compounds that form hydrogen by a chemical reaction, and wherein preferably the compounds forming the combination of compounds are physically and/or spatially separated from each other.
5. Composition or kit of compositions according to any one of aspects 1 to 4, wherein A, the compound or a combination of compounds capable of generating molecular hydrogen (H₂) and comprising at least one of zinc, magnesium, and iron, and B, the one or more active ingredients selected from the group consisting of compounds recited in aspect 1, are physically and/or spatially separated from each other.
6. Composition or kit of compositions according to any one of aspects 1 to 5, wherein at least one of B, the one or more active ingredients selected from the group consisting of compounds recited in aspect 1, is present in encapsulated form, and/or wherein a compound or at least one of a combination of compounds capable of generating molecular hydrogen (H2) and comprising at least one of calcium, zinc, magnesium, and iron, is present in encapsulated form.
7. Composition or kit of compositions according to any one of aspects 1 to 6, wherein the at least one of calcium, magnesium, iron and zinc comprised in A is present in micronized form having an average particle size of 1000 µm or less, and/or wherein at least one of the one or more active ingredients B is present in micronized form having an average particle size of 1000 µm or less.
8. Composition or kit of compositions according to any one of aspects 1 to 7, wherein the one or more active ingredient B selected from the group of compounds recited in aspect 1 is present in lyophilized form.
9. Composition according to any one of aspects 1 to 8, wherein the amount of Component A is selected such as to generate 0.1 mg of hydrogen or more, relative to 1g of the composition or kit of compositions.
10. Composition or kit of compositions according to any one of aspects 1 to 9, wherein the composition or kit of compositions is selected from the group consisting of
   (i) a single composition in the form of an effervescent tablet or effervescent powder, or a kit of compositions each being in the form of an effervescent tablet or effervescent powder; and
   (ii) a single composition in the form of a solid or semi-solid preparation, preferably a powder, ointment, gel, or cream, or a kit of compositions each being in the form of a solid or semi-solid preparation, such as a powder to tablet
11. Composition or kit of compositions according to any one of aspects 1 to 10, which is for use in a method of treatment or prevention of the human body, preferably for use in a method of treatment or prevention of age-associated diseases, treatment or prevention of diseases associated with oxidative stress, e.g. cancer, or cardiovascular diseases.
12. Non-medical use of the composition or kit of compositions according to any one of aspects 1 to 10, or a product obtained therefrom by adding water or an aqueous solution, for cosmetic applications or as food supplement.
13. Composition or kit of compositions according to aspect 11 or use of the composition or kit of compositions according to aspect 12, wherein the composition or kit of compositions takes the form of a tablet, capsule, powder, effervescent tablet, ointment, gel, creme or solution.
14. Cosmetic preparation or food supplement, including a composition or kit of compositions according to any one of aspects 1 to 10, or being obtained therefrom by adding water or an aqueous solution.
15. Pharmaceutical preparation for topical or oral administration comprising a composition or kit of compositions according to any one of aspects 1 to 10 or being obtained therefrom by adding water or an aqueous solution.

The present application is not limited to the above aspects, and further and preferred features of the present invention will become apparent in view of the following detailed description.

### DEFINITIONS

In the present invention, the term "comprising" is used in an open-ended manner, indicating the necessary presence of the following feature, component, et cetera. However, the term "comprising" does not exclude the presence of further components or features. Nonetheless, the term "comprising" also encompasses the meaning "consists of", thereby excluding the presence of further components.

The term "composition" is used to denote a substance or agglomeration of matter wherein the respective components form a substantially continuous phase. Here, the term "substantially continuous phase" means that there is no large spacing between the components, yet smaller spacings may be present, such as pores in powder or compressed powder tablets. The composition can be formed by particles (such as a powder), or can be in the shape of a solid body (such as a tablet or effervescent tablet). A composition can also be in semi-solid form, such as a paste or cream, but is typically in solid form, e.g. tablet or powder.

The term "kit of compositions" is used to denote a kit of at least 2 compositions as defined above. The compositions of the kit are generally separated from each other. An example of a kit of compositions is a kit of 2 or more containers, such as sachets or blisters, each containing a powder or tablet. The 2 or more containers may be part of the same physical entity (such as a blister containing 2 different types of powders or tablets), but may also be present separately.

The terms "one or more" and "at least one" are used synonymously, and noted that at least 1 of the following compounds is present. However, in the list of compounds, also 2 or more, 3 or more, 4 or more or all of the respective compounds may be present.

The term "average particle size" refers to the median (D50) on a number basis, as determined by a laser light scattering technique using e.g. a HORIBA particle size analyser.

### DETAILED DESCRIPTION OF THE INVENTION

The following will provide a detailed description of the components present in the compositions, respectively the kit of compositions of the present invention.

### A Compound or combination of compounds capable of generating molecular hydrogen (H₂) and comprising at least one of calcium, zinc, magnesium, and iron

In order to provide for the generation of hydrogen, the composition (or kit of compositions) of the present invention needs to comprise a compound that is capable of generating molecular hydrogen. In the present invention, this compound can be metallic, and in this case it comprises at least one of metallic calcium, metallic zinc, metallic magnesium and metallic iron. Here, the term "metallic" denotes that of the respective element is present in the oxidation state 0, i.e. not in ionized form.

Calcium and magnesium in their metallic form are known to be capable of generating hydrogen upon contact with water. The generation of hydrogen can be facilitated by providing of a calcium and magnesium in particulate form, preferably having an average particle size of 1000 nm or less. The average particle size can preferably be 800 nm or less, such as 500 nm or less or 400 nm or less.

Upon contact with water, hydrogen gas is generated, and the magnesium or calcium is converted to an ionised species, i.e. to the respective calcium or magnesium hydroxide. This reaction products can also be beneficial to a user or patient, given that calcium and magnesium are known to be biologically active elements.

Iron and zinc in metallic form are also able to generate molecular hydrogen, yet require an acidic pH (i.e. less than 7) for the reaction to proceed. For this reason, iron and zinc are preferably used in a combination of compounds that together are able to generate molecular hydrogen, one of the combination of compounds being metallic iron or zinc, the other one being an organic acid, such as citric acid, malic acid, maleic acid, malonic acid, succinic acid, tartaric acid, acetic acid, fumaric acid, or ascorbic acid (vitamin C). In such a case, the protons dissociated from the respective acids react with the zinc or iron by a redox reaction, forming molecular hydrogen and the respective iron and zinc salts. If desired, also an acidic buffer may be used that is able to yield the required pH upon adding water, e.g. based on acetic acid and its salts or phosphoric acid and its salts. Such an organic acid can of course also be used together with metallic magnesium or calcium, as it may facilitate the hydrogen formation reaction.

The respective iron and zinc salts resulting from the reaction forming molecular hydrogen can also be biologically beneficial, given that iron and zinc are biologically essential elements and play a role for instance in the regulation of the immune system. Also magnesium salts and calcium salts can be biologically beneficial. Furthermore, iron generally is preferably in the oxidation state +2 to have a biological action, as compared to iron in the oxidation state +3. If therefore a patient or user is to be provided with biologically active iron, the present invention can provide an additional benefit due to the hydrogen preventing oxidation to the state +3.

As will also be discussed below, prior to use by a patient or user, the composition (or the kit of compositions) may have to be combined with water to obtain a product ready for application, e.g. in the case of an effervescent tablet. In view of the above, the compound capable of generating molecular hydrogen may in this case be any of metallic calcium, metallic zinc, metallic magnesium, and metallic iron. Alternatively, the composition or kit of compositions may comprise a salt of any of calcium, zinc, magnesium and iron capable of generating hydrogen, such as the respective hydrides. However, since hydrides can be highly reactive, the use of metallic calcium, zinc, magnesium or iron may be preferred.

In particular for metallic zinc and metallic iron, the component A is however preferably a combination of compounds capable of generating molecular hydrogen, and this combination may comprise (or consist of) metallic iron and/or zinc and an organic acid. Of course, also metallic calcium and magnesium may be combined with an organic acid to form a combination of compounds capable of generating hydrogen. Put differently, in one embodiment a combination of compounds capable of generating molecular hydrogen by a chemical reaction can be defined as a combination of at least one of metallic iron, metallic zinc, metallic calcium or metallic magnesium with an organic acid. Here, the organic acid is preferably not a fatty acid, and suitable examples include citric acid, malic acid, maleic acid, malonic acid, succinic acid, tartaric acid, acetic acid, fumaric acid, or ascorbic acid. It is also possible to replace such an acid by an acidic buffer that causes a suitable pH upon addition of water or an aqueous solution.

Alternatively, water may already be a part of the composition (or the kit of compositions), and in this case water may be one of the combination of compounds capable of generating molecular hydrogen (e.g. in combination with magnesium or calcium). In such an embodiment, it is however necessary to prevent premature reaction between the water and the zinc, iron, calcium or magnesium (e.g. metallic magnesium or calcium, or hydrides thereof), so that the water needs to be encapsulated or otherwise be immobilised such as to avoid the reaction with the magnesium or calcium, e.g. in metallic or hydride form. Such an embodiment may be particularly preferable if the final product is not in aqueous solution (such as a drinkable preparation obtained by dissolving an effervescent tablet), but should have viscous or pasty consistency, such as an ointment, cream, or gel. Including the water in separated form would in this case for the benefit that no water is required for obtaining the composition ready for use. Yet, it is of course also possible to generate hydrogen with water that is added from an external source at the time of preparation by medical staff or the user.

In this and other embodiments wherein the component A is formed by a combination of compounds capable of generating molecular hydrogen, it may be preferable to ensure that no premature reaction between the compounds occurs. This can be ensured by providing the respective compounds forming the combination in different compositions of the kit of compositions, as the respective compositions forming the kit are typically combined by medical staff or a user immediately prior to use, i.e. at a point in time where the reaction is desired. Alternatively, the compounds forming the combination of compounds capable of generating molecular hydrogen may be spatially and/or physically separated from each other in a single composition. A spatial separation in a single composition can be achieved by forming for instance a tablet having discrete regions comprising the step compounds separately (such as bi- or multilayer (effervescent) tablet). A physical separation can be achieved by encapsulating one or both of the compounds forming the combination of components capable of forming molecular hydrogen. The capsule material is in this case preferably water-soluble, e.g. formed of PEG or a liposome. Alternatively, the metal (typically in the form of particles) may be coated with a water soluble or non-water-soluble compound, such as a fatty acid. Suitable methods for encapsulating or coating the respective compounds are known to a skilled person, and are often used in the cosmetic/personal hygiene fields, e.g. for encapsulating skin-beneficial agents.

It is generally sufficient if the component A comprises one element selected from iron, calcium, zinc and magnesium, which is preferably metallic. However, two or more of these may be used. As the reaction of calcium with water may be vigorous, the element is preferably selected from iron, zinc and magnesium. In one embodiment, two or more of iron, zinc and magnesium can be used, such as a combination of iron and magnesium, a combination of zinc and magnesium, a combination of iron and zinc, or a combination of iron, zinc and magnesium. In these cases, each of these can be present in metallic form, and calcium and magnesium may alternatively or additionally be present in ionised form allowing for the formation of hydrogen, e.g. as hydrides. However, also in particular metallic or ionised magnesium may be used solely.

### B Active Ingredient

The composition or kit of compositions according to the present invention comprises besides the compound (or combination of components) capable of generating molecular hydrogen, A, one or more active ingredients B. The active ingredient is selected from the group consisting of nicotinamide mononucleotide (NMN), glutathione, hyaluronic acid and salts thereof, polyunsaturated fatty acids, salts and C1-C6 alkyl esters thereof, mono- and polyunsatured oils and fats, medium-chain triglycerides, fruits and vegetables or extracts thereof, coenzyme Q10, aloe vera and vitamins other than vitamin C. Of these, NMN, glutathione and hyaluronic acid and salts thereof are preferred, and NMN is more preferred. Also, one of these preferred ingredients, in particular NMN, can be combined with a fruit or vegetable extract, as described below. Herein, NMN can be present in any form, including lyophilized and liposomal.

Nicotinamide mononucleotide (NMN) and glutathione are known to have anti-ageing properties and to be beneficial in reducing oxidative stress. These compounds can be used in any of the compositions (or kit of compositions) of the present invention, i.e. in all of medical and cosmetic applications, both to be administered orally or topically.

Hyaluronic acid and salts thereof are known to provide for multiple benefits, e.g. also in the treatment of age-related diseases. These compounds can be used in any of the compositions (or kit of compositions) of the present invention, i.e. in all of medical and cosmetic applications, both to be administered orally or topically.

Polyunsaturated fatty acids, such as DHA and EHA, and salts and C1-C6 alkyl esters thereof (e.g. ethyl ester), as well as mono- and polyunsatured oils and fats (e.g. cacao butter) can also be used for preparations to be ingested by the user or patient, but may also provide benefit in topical preparations (which may be cosmetic or pharmaceutical). Given that these compounds are typically taken for their benefit in preventing cardiovascular diseases and/or reducing oxidative stress, these compounds are particularly suitable for preparations that are to be ingested.

Medium-chain triglycerides (MCT) are glycerine triesters with medium length saturated acids having 6 to 12 carbon atoms. These are typically mixed triesters, i.e. esters of glycerine with two or three different species selected from saturated C6 - to C12 acids, such as esters of glycerine with octanoic acid (caprylic acid, C8), decanoic acid (capric acid, C10) and /or dodecanoic acid. These compounds are believed to contribute to a healthy diet and to facilitate weight loss, and are therefore typically used for compositions that shall yield a digestible formulation, such as powders or (effervescent) tablets.

Coenzyme Q10 and Aloe Vera are known to provide for skin benefits, and are therefore preferable for compositions that shall yield a product for topical application. However, they may also be used in compositions for preparing products for oral administration.

Fruits, vegetables and extracts thereof are known to contribute to a healthy diet, and are therefore particularly suitable for compositions for use as food supplement. However, they can also be used in cosmetics, e.g. for topical application. Examples of vegetables, fruits and extracts thereof include an extract comprising oligomeric proanthocyanidins (OPC), such as an extract of grape seeds, an extract containing trimethyl glycine (TMG), such as an extract of sugar beet, an extract containing berberine, e.g. an extract from barberry, an extract comprising one or more polyphenols, such as resveratrol, obtained from e.g. green tea or grapes, and others. The fruits, vegetables and extracts thereof can be used in any form, but is particularly preferably used in lyophilized form, as will also be discussed below.

The active ingredient B can be one or more of the components indicated above. In certain embodiments, it may be preferable to spatially and/or physically distance the active ingredient from the compound or combination of components A capable of generating molecular hydrogen, e.g. in order to avoid an undesired reaction prior to preparation of the final product. This can be achieved in a similar manner as outlined above. For instance, in a single composition, different portions (layers) of a tablet may be provided, of which one comprises of a component A for generating molecular hydrogen, and another one comprises of a component B. Alternatively, the active ingredient may be encapsulated, e.g. by the liposomal coating or capsule, such as liposomal NMN. Of course, also encapsulation in a water-soluble polymeric material such as polyethylene glycol (PEG) or coating with a lipophilic material, such as fat, wax or a fatty acid, is conceivable.

In these and other embodiments of the present invention, the active ingredient of component B may be present in liposomal and/or lyophilised form. It is considered that this is preferable in order to avoid decay of the active ingredient upon storage, and may also avoid an undesirable reaction with the component A if present in the same composition and/or not spatially or physically separated from the component B. Suitable techniques for lyophilising the active ingredients and/or providing them in liposomal form are well known to a skilled person. Liposomal products, including active ingredients B, are also commercially available, e.g. under the tradename LipoCellTech^{™}. Information on the properties and manufacture of liposomal products can be found in the literature, such as in "Encapsulation of Food Ingredients Using Nanoliposome Technology" by M. Reza Mozafari et al., International Journal of Food Properties Volume 11, 2008 - Issue 4 or Y. Shoji et al, Journal of Drug Targeting, Vol. 12 (6), 2004, pages 385-391. See also *"*Health Effects of Dietory Phospholipids " by D. Kullenberg et al, Lipids in Health and Disease 11:3, 2012.

The present invention thus includes embodiments wherein the active ingredient is one or more selected from liposomal and/or lyophilized glutathione, liposomal and/or lyophilized NMN, liposomal and/or lyophilized hyaluronic acid or salts thereof, and a fruit or vegetable extract comprising one or more of OPC, TMG, berberine and polyphenols, preferably in lyophilized form. In a preferred aspect, the composition of the present invention or the kit of compositions comprises liposomal and/or lyophilized NMN, which may be combined with one or more selected from liposomal and/or lyophilized glutathione, liposomal and/or lyophilized hyaluronic acid or salts thereof, and a fruit or vegetable extract comprising one or more of OPC, TMG, berberine and polyphenols, preferably in lyophilized form . Here, "liposomal and lyophilized" denotes a compound B that is lyophilized and that is provided with a liposomal coating, preferably after the active ingredient B has been lyophilized.

A liposomal active ingredient B may be liquid or dry form. A dry form, such as in powder form, can be preferable, as this may facilitate storage, handling and manufacture. A dry form may also be prepared without having to rely on higher temperatures, high pressures or the use of potentially hazardous chemicals.

Further, without wishing to be bound by theory, it is believed that providing the active ingredient B in liposomal form may not only provide for greater protection of the active ingredient against decay, e.g. by oxidation with molecular oxygen from the surrounding atmosphere, and may thus allow realizing an improved shelf life, but may additionally or alternatively provide for a greater bioavailability. This is because active ingredients in liposomal form are believed to be capable of penetrating a cell membrane easier than a non-liposomal active ingredient, as the liposomal coating facilitates penetration of the cell wall. Further, it is considered that in particular in combination with molecular hydrogen, which is also capable of penetrating the cell wall easily due to its small size, a particular high benefit to a user or patient can be provided. The combination of molecular hydrogen and a liposomal active ingredient B may thus open the possibility of obtaining a synergistic or improved effect, i.e. an effect that is not, or is not to this extent, observed with only the liposomal active ingredient B or molecular hydrogen when administered separately.

Also, dry (powder) liposomal active ingredients B may exhibit greater bioavailability than their liquid counterparts.

Additionally, using a liposomal active ingredient may be preferable in order to mask and otherwise unpleasant flavour or smell.

### C Additives

Besides the essential components A and B, the composition or kit of compositions of the present invention may comprise optional additives as well known to a skilled person. In one embodiment, the additive may be selected from the group consisting of antioxidants, fillers, buffers, preferably a carbonate/bicarbonate buffer, pH adjusting agents, flavours, sugars, alkali earth salts of organic acids, and vitamin C.

Out of these additives, the presence of a buffer is preferable, given that the reaction for the generation of hydrogen generally leads to a change in pH. The buffer is preferably selected and used in such an amount that upon complete generation of hydrogen the pH of the resulting product formed from the composition (or kit of compositions) of the present invention is within the range of from 4.0 to 8.0, preferably 5.5 to 7.5.

If the product is ultimately designed for ingestion, certain types of flavours (e.g. lemon, strawberry, cranberry, cherry or orange) and/or sugars may be used. Also, other types of ingredients having a biological action, such as taurine or caffeine, or a (green) tea extract, may be used.

In particular for applications as food supplement, also inorganic salts of organic acids may be used, in particular salts of magnesium or calcium. A preferred salt includes magnesium citrate and magnesium malate, due to their high bioavailability.

Further examples of optional additives include starch, such as corn starch, peptides, and similar. The type of additive is also determined by the desired end use of the product prepared by using the composition (or kit of compositions) of the present invention. For instance, if the final product shall be used as a cosmetic for topical application (such as cream or gel), typical additives such as an emollient (such as natural and artificial fats, natural and artificial waxes, and polyethylene glycols) or glycerine may be present.

Further examples of optional additives include particles known to exhibit biological action, such as an antibacterial action. Such particles include colloidal silver.

### Composition or Kit of Compositions

The components above may be mixed to form a single composition, e.g. in powder form, and this product may then be compressed to form a tablet (which, depending on the choice of components, may be an effervescent tablet). A tablet forming a single composition may also be a multilayer or multi-sectional tablet wherein the components are spatially separated from each other.

A kit of compositions (i.e. a kit of 2 tablets, 2 powders or a tablet and a powder) has the advantage that the components may be separated from each other more easily without having to rely on complicated manufacturing techniques. Furthermore, separation between the components may be more reliable in case of 2 or more separate compositions (forming a kit) being provided.

In a kit of compositions, one of the compositions may comprise a component A, and the other one may comprise a component B. In the embodiment wherein the component A is formed by a combination of compounds that together are capable of generating molecular hydrogen by a chemical reaction (e.g. a combination of zinc and/or iron with an organic acid), one of these compounds may be present in one composition, and the other one may be present in another composition forming a part of the kit of compositions. For instance, one of the compositions forming the kit of compositions may contain the active ingredient in combination with an organic acid (such as citric acid), and another one of the compositions forming a kit of compositions may contain metallic iron, magnesium and/or zinc.

### Preparation of cosmetic or pharmaceutical product or food supplement

In the case of a food supplement, the composition of the present invention may be used as it is, by adding the composition to the food. In case of a tablet, it also can be directly ingested.

In the case of a gel-like or creamy cosmetic product for topical application or a liquid preparation for use as cosmetic or for medical purposes, or as a food supplement, it may however be required to combine the composition or kit of compositions with water or an aqueous solution. This will then allow the component A to begin with the generation of hydrogen. Of note in this respect is that hydrogen is volatile and may diffuse out of a composition over time. As such, by preparing the final product immediately prior to use, a high hydrogen concentration in the product can be ensured.

The present invention therefore also includes a method for the preparation of a pharmaceutical or cosmetic product or a food supplement, the method being a method as defined below:
I. A method wherein a composition of the present invention is combined with water or an aqueous solution to prepare a product ready for topical or oral administration for pharmaceutical or cosmetic purposes, or as food supplement;
II. A method wherein the compositions forming the kit of compositions of the present invention are combined, optionally together with water or an aqueous solution, to prepare a product ready for topical or oral administration for pharmaceutical or cosmetic purposes.

The present invention furthermore pertains to a pharmaceutical or cosmetic product or a food supplement obtained by a method as defined above, which pharmaceutical or cosmetic product or food supplement is in liquid or semi-solid form, such as a gel, lotion, creme or ointment.

### EXAMPLE

A tablet resembling a composition in accordance with the present invention can be prepared by mixing and tabletting a formulation consisting of

| | |
|---|---|
| Glucose | 1170 parts by mass |
| Magnesium malate | 600 parts by mass |
| Hyaluronic acid, sodium salt | 120 parts by mass |
| Liposomal NMN | 150 parts by mass |
| Sodium bicarbonate/ | |
| Sodium hydrogen carbonate buffer | 50 parts by mass |
| Citric acid | 20 parts by mass |
| Ascorbic acid | 20 parts by mass |
| Magnesium Stearate | 5 parts by mass |
| Magnesium particles | 25 parts by mass |

The tablet can be mixed with sufficient water to generate molecular hydrogen.

## Claims

1. A composition or kit of compositions comprising, either in a single composition or in distinct compositions of a kit of compositions,
A a compound or a combination of compounds capable of generating molecular hydrogen (H₂) and comprising at least one of calcium, zinc, magnesium, and iron, and
B one or more active ingredients selected from the group consisting of nicotinamide mononucleotide (NMN), glutathione, hyaluronic acid and salts thereof, polyunsaturated fatty acids, salts and C1-C6 alkyl esters thereof, mono- and polyunsatured oils and fats, medium-chain triglycerides, fruits and vegetables and extracts thereof, coenzyme Q10, aloe vera and vitamins other than vitamin C.

2. Composition or kit of compositions according to claim 1 or 2, wherein A, the compound or combination of compounds capable of generating molecular hydrogen (H₂) and comprising at least one of calcium, zinc, magnesium, and iron, comprises a compound that generates molecular hydrogen upon contact with water, or comprises a combination of compounds that by reaction with each other generate molecular hydrogen.

3. Composition or kit of compositions according to any one of claims 1 to 3, which further comprises
C one or more additives, preferably selected from the group consisting of antioxidants, fillers, such as starch, buffers, preferably a carbonate/bicarbonate buffer, pH adjusting agents, flavours, sugars, earth alkaline salts of organic acids, and vitamin C, more preferably a salt of magnesium and an organic acid, such as magnesium malate.

4. Composition or kit of compositions according to any one of claims 1 to 3, wherein A, the compound or a combination of compounds capable of generating molecular hydrogen (H₂) and comprising at least one of calcium, zinc, magnesium, and iron, is a combination of compounds that form hydrogen by a chemical reaction, and wherein preferably the compounds forming the combination of compounds are physically and/or spatially separated from each other.

5. Composition or kit of compositions according to any one of claims 1 to 4, wherein A, the compound or a combination of compounds capable of generating molecular hydrogen (H₂) and comprising at least one of zinc, magnesium, and iron, and B, the one or more active ingredients selected from the group consisting of compounds recited in claim 1, are physically and/or spatially separated from each other.

6. Composition or kit of compositions according to any one of claims 1 to 5, wherein at least one of B, the one or more active ingredients selected from the group consisting of compounds recited in claim 1, is present in encapsulated form, and/or wherein a compound or at least one of a combination of compounds capable of generating molecular hydrogen (H2) and comprising at least one of calcium, zinc, magnesium, and iron, is present in encapsulated form.

7. Composition or kit of compositions according to any one of claims 1 to 6, wherein the at least one of calcium, magnesium, iron and zinc comprised in A is present in metallic and micronized form having an average particle size of 1000 µm or less, and/or wherein at least one of the one or more active ingredients B is present in micronized form having an average particle size of 1000 µm or less.

8. Composition or kit of compositions according to any one of claims 1 to 7, wherein the one or more active ingredient B selected from the group of compounds recited in claim 1 is present in lyophilized form and/or in liposomal form.

9. Composition according to any one of claims 1 to 8, wherein the amount of Component A is selected such as to generate 0.1 mg of hydrogen or more, relative to 1g of the composition or kit of compositions.

10. Composition or kit of compositions according to any one of claims 1 to 9, wherein the composition or kit of compositions is selected from the group consisting of
(iii) a single composition in the form of an effervescent tablet or effervescent powder, or a kit of compositions each being in the form of an effervescent tablet or effervescent powder; and
(iv) a single composition in the form of a solid or semi-solid preparation, preferably a powder, ointment, gel, or cream, or a kit of compositions each being in the form of a solid or semi-solid preparation, such as a powder to tablet

11. Composition or kit of compositions according to any one of claims 1 to 10, which is for use in a method of treatment or prevention of the human body, preferably for use in a method of treatment or prevention of age-associated diseases, treatment or prevention of diseases associated with oxidative stress, e.g. cancer, or cardiovascular diseases.

12. Non-medical use of the composition or kit of compositions according to any one of claims 1 to 10, or a product obtained therefrom by adding water or an aqueous solution, for cosmetic applications or as food supplement.

13. Composition or kit of compositions according to claim 11 or use of the composition or kit of compositions according to claim 12, wherein the composition or kit of compositions takes the form of a tablet, capsule, powder, effervescent tablet, ointment, gel, creme or solution.

14. Cosmetic preparation or food supplement, including a composition or kit of compositions according to any one of claims 1 to 10, or being obtained therefrom by adding water or an aqueous solution.

15. Pharmaceutical preparation for topical or oral administration comprising a composition or kit of compositions according to any one of claims 1 to 10 or being obtained therefrom by adding water or an aqueous solution.
